(19) Europäisches Patentamt
European Patent Office
Office européen des brevets

(11) **EP 2 110 657 A1**

(12) **DEMANDE DE BREVET EUROPEEN**

(43) Date de publication:
**21.10.2009 Bulletin 2009/43**

(51) Int Cl.:
*G01N 21/03* (2006.01)      *G01N 21/47* (2006.01)
*G01N 21/64* (2006.01)      *A61B 5/00* (2006.01)

(21) Numéro de dépôt: **09158148.8**

(22) Date de dépôt: **17.04.2009**

(84) Etats contractants désignés:
**AT BE BG CH CY CZ DE DK EE ES FI FR GB GR
HR HU IE IS IT LI LT LU LV MC MK MT NL NO PL
PT RO SE SI SK TR**

(30) Priorité: **18.04.2008 FR 0852633**

(71) Demandeur: **Commissariat à l'Energie Atomique
75015 Paris (FR)**

(72) Inventeurs:
• **Da Silva, Anabella
13004 MARSEILLE (FR)**

• **Rizo, Philippe
38700, La Tronche (FR)**
• **Berger, Michel
38640, Claix (FR)**
• **Guyon, Laurent
38100, Grenoble (FR)**

(74) Mandataire: **de Beaumont, Michel
1bis, rue Champollion
38000 Grenoble (FR)**

(54) **Dispositif optique pour l'analyse d'un milieu diffusant maintenu par un support**

(57) L'invention concerne un dispositif optique (49) destiné à l'analyse d'un milieu diffusant (18), comprenant au moins une source lumineuse (14) à distance du milieu diffusant et adaptée à fournir un faisceau lumineux incident (15) destiné à éclairer le milieu diffusant ; au moins un capteur (16) adapté à détecter un rayonnement émis par le milieu diffusant ; un support (50 ; 56) du milieu diffusant au moins partiellement non absorbant pour le faisceau lumineux incident et le rayonnement diffus. Tout ou partie du support est constitué d'un matériau diffusant dont le coefficient de diffusion réduit est supérieur à 0,1 cm$^{-1}$ et inférieur à 700 cm$^{-1}$

Fig 4

EP 2 110 657 A1

**Description**

Domaine de l'invention

La présente invention concerne un dispositif optique pour l'analyse d'un milieu diffusant maintenu par un support.

Exposé de l'art antérieur

Un domaine de l'imagerie optique concerne l'analyse d'un milieu diffusant, par exemple l'analyse de tissus biologiques.

De façon générale, le milieu diffusant est soumis à un faisceau lumineux incident. Pour certaines applications, par exemple en tomographie optique diffusive, la longueur d'onde du faisceau incident est dans le spectre visible, de façon classique dans le rouge ou dans le proche infrarouge, ce qui correspond à la plage de longueurs d'onde où l'absorption des tissus biologiques est minimale. Le faisceau lumineux incident diffuse dans le milieu diffusant et le rayonnement lumineux diffus qui s'échappe du milieu diffusant est détecté par un capteur, par exemple une caméra. Les images fournies par le capteur sont analysées pour déterminer les propriétés optiques du milieu diffusant, par exemple pour déterminer la distribution des propriétés optiques de diffusion et/ou d'absorption du milieu diffusant, par exemple pour détecter la présence d'une zone anormalement absorbante et/ou diffusante.

Pour d'autres applications, par exemple en tomographie optique diffusive de fluorescence, on dispose dans le milieu diffusant des marqueurs fluorescents spécifiques qui tendent à se concentrer dans des zones d'intérêt. Lorsqu'ils sont éclairés par le faisceau lumineux incident, les marqueurs émettent un rayonnement à une longueur d'onde distincte de celle du faisceau incident. Ce rayonnement diffuse dans le milieu diffusant et est détecté et analysé pour déterminer les propriétés des marqueurs (par exemple, la localisation et la concentration locale des marqueurs).

Dans le domaine de l'imagerie médicale, les techniques d'imagerie optique offrent, par exemple pour la détection et la localisation de tumeurs cancéreuses, une alternative aux techniques d'imagerie classiques telles que la radiographie et tomographie par rayons X, la tomographie par émission de positons, l'imagerie par résonance magnétique, etc.

Pour certains dispositifs optiques, la source lumineuse est en contact direct avec le milieu diffusant. A titre d'exemple, le faisceau lumineux incident est guidé jusqu'au milieu diffusant par des fibres optiques. Le rayonnement diffus émis par le milieu diffusant peut également être récupéré par des fibres optiques et guidé jusqu'au capteur. Un avantage est que l'environnement du milieu diffusant, en particulier le support maintenant le milieu diffusant, ne perturbe pas les mesures. Toutefois, ces dispositifs optiques sont généralement encombrants car la précision accessible de la distribution des propriétés optiques dépend du nombre de mesures indépendantes pouvant être effectuées, c'est-à-dire du nombre de fibres optiques utilisées.

Pour d'autres dispositifs optiques, la source lumineuse est à distance du milieu diffusant. La structure du dispositif optique peut alors être simplifiée. Le support maintenant le milieu diffusant est généralement conçu de façon à perturber le moins possible la propagation des rayons lumineux. Pour ce faire, le support est constitué d'un matériau non absorbant et non diffusant pour la plage de longueurs d'onde utile.

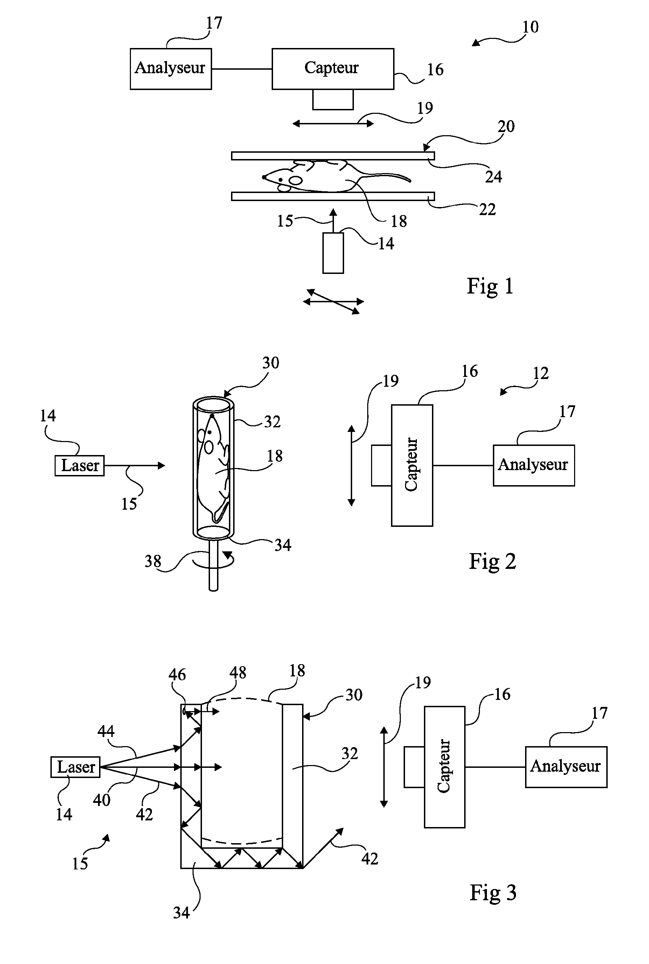

Les figures 1 et 2 représentent deux exemples de réalisation de dispositifs optiques pour lesquels la source lumineuse est à distance du milieu diffusant. Chaque dispositif optique 10, 12 comprend une source lumineuse 14, par exemple une diode laser fournissant un faisceau lumineux monochromatique 15 dont la longueur d'onde est, par exemple, de 670 nanomètres, et un capteur d'images 16 adapté à la détection d'un rayonnement diffus. Un outil d'analyse 17 est relié au capteur 16 et réalise un traitement des images acquises par le capteur 16. Un milieu diffusant à analyser 18, par exemple une souris, est disposé de façon à recevoir le faisceau lumineux incident 15 fourni par la source lumineuse 14. Une lentille 19 est disposée entre le milieu diffusant 18 et le capteur 16. L'axe du capteur 16 peut ne pas être parallèle au faisceau incident 15. Selon une variante, les images fournies par le capteur 16 peuvent être enregistrées par l'outil 17 puis analysées ultérieurement au moyen d'un autre ordinateur.

Pour le dispositif 10 représenté en figure 1, le milieu 18 à analyser est maintenu par un support 20 comprenant une plaque d'entrée 22 située entre le milieu 18 et la source lumineuse 14 et une plaque 24 de sortie située entre le milieu 18 et le capteur 16. Les plaques 22, 24 sont transparentes et parallèles. A titre d'exemple, le support 20 peut être fixe et la source lumineuse 14 peut se déplacer par rapport au support 20. Pour le dispositif 12 représenté en figure 2, le milieu 18 à analyser est maintenu par un support 30 correspondant à un tube transparent comprenant une paroi latérale cylindrique 32 et un fond plan 34. A titre d'exemple, le support 20 est monté sur un bras rotatif 38, la source 14 pouvant être fixe.

Le support 20, 30 est de façon générale en plastique ou en verre. Toutefois, l'utilisation de tels supports peut entraîner la formation d'artefacts sur les images fournies par le capteur 16. Ces artefacts peuvent gêner voire empêcher l'analyse du milieu diffusant 18.

Résumé

La présente invention vise un dispositif optique pour l'analyse d'un milieu diffusant maintenu par un support comprenant une source lumineuse et un capteur, la source lumineuse étant à distance du milieu à analyser, le support permettant de réduire, voire de supprimer, la formation d'artefacts sur les images acquises par le capteur.
Une autre caractéristique du support est qu'il peut être fabriqué simplement.
Une autre caractéristique est que les propriétés optiques du support varient peu dans le temps.
Ainsi, un mode de réalisation de la présente invention prévoit un dispositif optique destiné à l'analyse d'un milieu diffusant. Le dispositif comprend au moins une source lumineuse à distance du milieu diffusant et adaptée à fournir un faisceau lumineux incident destiné à éclairer le milieu diffusant ; au moins un capteur adapté à détecter un rayonnement émis par le milieu diffusant ; un support du milieu diffusant au moins partiellement non absorbant pour le faisceau lumineux incident et le rayonnement diffus. Tout ou partie du support est constitué d'un matériau diffusant dont le coefficient de diffusion réduit est supérieur à 0,1 cm$^{-1}$ et inférieur à 700 cm$^{-1}$.
Selon un mode de réalisation de l'invention, le coefficient de diffusion réduit du support est compris entre 1 et 700 cm$^{-1}$.
Selon un mode de réalisation de l'invention, ledit matériau a un coefficient d'absorption compris entre 0.01 et 300 cm$^{-1}$.
Selon un mode de réalisation de l'invention, le support comprend au moins une paroi destinée à être située entre la source et le milieu diffusant et/ou entre le milieu diffusant et le capteur.
Selon un mode de réalisation de l'invention, le support a la forme d'un berceau destiné à recevoir le milieu diffusant.
Un mode de réalisation de l'invention prévoit un support adapté au dispositif optique décrit précédemment.
Un mode de réalisation de l'invention prévoit une utilisation du dispositif optique décrit précédemment, pour réaliser par tomographie une cartographie à trois dimensions du coefficient d'absorption et/ou du coefficient de diffusion réduit du milieu diffusant.
Un mode de réalisation de l'invention prévoit une utilisation du dispositif optique décrit précédemment, le faisceau lumineux incident étant à une première longueur d'onde, l'utilisation consistant à placer dans le milieu diffusant des fluorophores sensibles à la première longueur d'onde et émettant le rayonnement diffus à une seconde longueur d'onde, le capteur fournissant des images utilisées pour déterminer des propriétés des fluorophores et/ou des propriétés du milieu diffusant.
Un mode de réalisation de l'invention prévoit un procédé de fabrication du support décrit précédemment. Le procédé comprend les étapes consistant à préparer une poudre d'un matériau diffusant ; à mélanger la poudre à une solution d'un matériau sensiblement non absorbant, non diffusant et durcissable ; et à injecter le mélange obtenu dans un moule pour former le support.
Selon un mode de réalisation de l'invention, le procédé comprend, en outre, l'étape consistant à mélanger une solution supplémentaire d'un matériau absorbant à la poudre ou à la solution.

Brève description des dessins

Ces objets, caractéristiques et avantages, ainsi que d'autres seront exposés en détail dans la description suivante d'un exemple de réalisation particulier faite à titre non-limitatif en relation avec les figures jointes parmi lesquelles :

les figures 1 et 2, précédemment décrites, représentent, de façon schématique, des exemples classiques de dispositifs optiques pour l'analyse d'un milieu diffusant ;
la figure 3 illustre de façon schématique certains artefacts pouvant être observés avec le dispositif de la figure 2 ;
les figures 4 et 5 représentent de façon schématique des premier et second exemples de dispositifs optiques selon un mode de réalisation de l'invention ; et
la figure 6 représente, sous la forme d'un schéma par blocs, un exemple de procédé de fabrication d'un support de dispositif optique selon un mode de réalisation de l'invention.

Description détaillée
Par souci de clarté, de mêmes éléments ont été désignés par de mêmes références aux différentes figures.
La Demanderesse est parvenue à la présente invention par une analyse des phénomènes à l'origine de la formation d'artefacts sur les images fournies par le capteur d'un dispositif optique pour l'analyse d'un milieu diffusant.
La figure 3 illustre de façon schématique certains phénomènes à l'origine de la formation d'artefacts pouvant être observés sur les images acquises par le capteur 16 du dispositif 12 de la figure 2. En figure 3, le support 30 est représenté en coupe latérale. Le faisceau lumineux incident 15 présente un angle d'ouverture et on a schématiquement représenté un rayon lumineux 40 du faisceau incident 15 sensiblement perpendiculaire à la paroi 32 et des rayons 42, 44 du faisceau incident 15 inclinés par rapport l'axe de la paroi 32. Le rayon lumineux 40 traverse sans perturbation le support 30 jusqu'à atteindre le milieu diffusant 18 à analyser.

Un premier type d'artefact résulte de rayons lumineux qui atteignent directement le capteur 16 sans avoir traversé le milieu à analyser. Ceci peut résulter d'un guidage optique de rayons inclinés 42, 44 par les parois du support 30. A titre d'exemple, les rayons inclinés 42 peuvent être guidés par la paroi latérale 32 et le fond 34 du support 30, puis s'échapper du support 30 et atteindre directement le capteur 16 sans avoir traversé le milieu diffusant 18. L'intensité des rayons lumineux 42 qui atteignent directement le capteur 16 peut être nettement supérieure à celle du rayonnement diffus. Le capteur 16 étant généralement adapté à la détection d'un rayonnement diffus de faible intensité, le fait que les rayons lumineux 42 atteignent directement le capteur 16 peut entraîner une saturation de ce dernier, ce qui rend inutilisables les images acquises. On pourrait considérer que les phénomènes décrits précédemment ne sont pas pénalisants pour la tomographie optique diffusive de fluorescence pour laquelle on détecte une image du rayonnement diffus émis par les marqueurs fluorescents à une longueur d'onde différente de la longueur d'onde du faisceau lumineux incident. Toutefois, la tomographie optique diffusive de fluorescence peut aussi utiliser l'image du rayonnement diffus émis par le milieu diffusant à la longueur d'onde du faisceau incident à des fins d'étalonnage.

Un second type d'artefact résulte de défauts 46 (inclusions, rayures, fissures, etc.) du support 30. En effet, lorsque les rayons lumineux 44, guidés optiquement par la paroi 32, atteignent le défaut 46, celui-ci peut émettre, par réflexion, réfraction ou diffraction un faisceau lumineux 48. Le défaut 46 est alors équivalent à une source lumineuse parasite contribuant au rayonnement émis par le milieu diffusant. Le second type d'artefacts peut être tout aussi pénalisant que le premier type d'artefact puisqu'il perturbe l'analyse des images détectées dans la mesure où les algorithmes de traitement des images fournies par le capteur 16 partent de l'hypothèse que les propriétés de l'illumination du milieu diffusant 18 sont bien connues, cette illumination pouvant résulter d'une source unique 14, de plusieurs sources lumineuses ponctuelles, de sources étendues, etc. En outre, la répartition de ces sources parasites n'étant pas connue, il s'avère difficile de les prendre en compte. Ce type d'artefact peut se produire aussi pour le dispositif optique 10 représenté en figure 1.

La présente invention prévoit d'utiliser un matériau diffusant pour réaliser le support du milieu à analyser optiquement. Les rayons lumineux du faisceau incident diffusant dès qu'ils progressent dans le support, on observe une atténuation très rapide des rayons lumineux qui tendent à être guidés optiquement par le support. Ceci permet de réduire fortement, voire d'annuler, la proportion de rayons lumineux susceptibles d'être guidés optiquement par le support jusqu'à sortir de celui-ci et atteindre directement le capteur d'images ou jusqu'à atteindre des défauts du support qui se comporteraient comme des sources lumineuses parasites. Le coefficient de diffusion du matériau constituant le support n'est toutefois pas choisi trop élevé pour ne pas entraîner une atténuation trop importante du rayonnement diffus émis par le milieu diffusant.

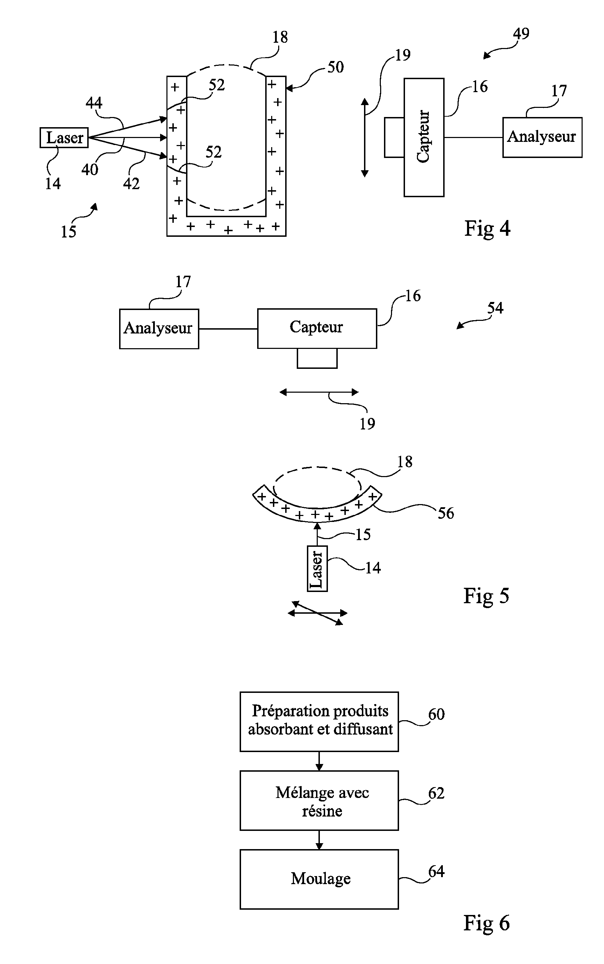

La figure 4 représente un premier exemple de réalisation d'un dispositif optique 49 selon l'invention. Le dispositif optique 49 comprend les mêmes éléments que le dispositif 12 représenté en figure 3 à la différence qu'il comporte un support 50 constitué d'un matériau diffusant. Les traits 52 désignent de façon schématique la région de diffusion du faisceau lumineux incident 15 atteignant le support 50. Dans l'exemple de réalisation représenté en figure 3, le support 50 a une forme tubulaire. Toutefois, le support 50 peut avoir une autre forme et, par exemple, comprendre deux plaques parallèles, comme pour le dispositif optique 10 représenté en figure 1.

Un tel support peut également être appliqué contre tout ou partie d'une surface d'un milieu diffusant à analyser, sans nécessairement contenir ni supporter l'objet diffusant à analyser dans une position donnée. L'objectif est alors d'aboutir à une diffusion de la lumière incidente produite par une source de lumière placée à distance du support, en procurant les avantages décrits ci après. Dans une telle configuration, le support pourra être appelé milieu intercalaire diffusant, l'adjectif intercalaire montrant bien une disposition entre une source de lumière externe et le milieu diffusant à analyser. Préférentiellement, ce support sera placé au contact de la surface du milieu diffusant, afin de constituer, avec le milieu diffusant, un unique ensemble diffusant.

La figure 5 représente un second exemple de réalisation d'un dispositif optique 54 selon l'invention. Le dispositif optique 56 comprend les mêmes éléments que le dispositif 10 représenté en figure 3 à la différence qu'il comporte un support 56 constitué d'un matériau diffusant et ayant la forme d'un berceau dans lequel est placé le milieu diffusant 18 à analyser. Dans le présent exemple de réalisation, la forme du support 56 est avantageusement adaptée au milieu diffusant 18. Ceci permet de réduire les possibilités de mouvement du milieu diffusant au cours de l'analyse, en particulier lorsque le milieu diffusant est un animal ou un être humain.

Le support étant lui-même un objet diffusant, il forme avec le milieu diffusant à analyser un système diffusant dont la surface externe est connue avec précision puisqu'elle correspond à la surface externe du support. Cette surface pouvant correspondre à une forme géométrique simple, la résolution des équations de propagation de la lumière dans les milieux diffusants peut être simplifiée.

Un autre avantage d'un tel support est de produire une diffusion de la lumière produite par le faisceau incident avant que cette dernière n'atteigne le milieu diffusant à analyser. La diffusion du faisceau incident dans le support entraîne un élargissement de la surface du milieu diffusant impactée par ce faisceau. Ainsi, un tel support permet de réduire l'intensité lumineuse surfacique à la surface du milieu diffusant. L'invention trouvera son intérêt lorsque le milieu diffusant est un tissu biologique et que la source lumineuse est une source Laser délivrant un faisceau intense. Dans un tel cas, le faisceau incident, sans diffusion préalable, pourrait générer des lésions, telles des brûlures, à la surface du milieu diffusant analysé. En présence du support objet de l'invention, le faisceau incident subit une diffusion avant d'atteindre la surface du milieu diffusant, réduisant ainsi le risque de lésion. Il est alors possible d'augmenter l'intensité du rayon incident, donc la quantité de lumière pénétrant dans le milieu diffusant, du fait de la diffusion de lumière en amont de la surface du milieu diffusant.

De façon avantageuse, pour améliorer le couplage optique entre le support et le milieu diffusant et donc faciliter la modélisation de la propagation des rayons lumineux dans le système diffusant composé du support diffusant et du milieu diffusant, les propriétés optiques de diffusion et, en outre, éventuellement d'absorption, du support peuvent être choisies proches de celles du milieu diffusant à analyser. Le coefficient de diffusion réduit $\mu'_S(sup)$ du matériau constituant le support est supérieur à 0,1 cm$^{-1}$ et est, de préférence, compris entre 1 et 700 cm$^{-1}$. Le coefficient d'absorption $\mu'_a(sup)$ du matériau constituant le support peut varier entre 0 et 300 cm$^{-1}$ et est, de préférence, compris entre 0,01 et 300 cm$^{-1}$.

Le support peut être constitué en totalité d'un matériau diffusant et éventuellement absorbant. Toutefois, seule une ou plusieurs parties du support peuvent être constituées du matériau diffusant, et éventuellement absorbant, le reste du support étant constitué d'un matériau sensiblement non absorbant et non diffusant. Les portions diffusantes sont alors avantageusement prévues à des emplacements adaptés du support pour éviter que des phénomènes de guidage optique n'entraînent la formation d'artefacts sur les images acquises par le capteur d'images. Le support selon la présente invention peut être utilisé avec un dispositif optique dans lequel le milieu diffusant baigne dans un liquide d'adaptation d'indices.

Le fait d'utiliser un matériau diffusant pour réaliser le support permet d'assurer que les propriétés optiques du support évoluent peu dans le temps. En effet, pour supprimer la formation d'artefacts sur les images fournies par le capteur du dispositif optique, on aurait pu utiliser un support non diffusant dont on aurait dépoli certaines faces. Toutefois, un tel traitement de surface peut être difficile à mettre en oeuvre, en particulier lorsque le support comprend des portions non planes. En outre, le dépolissage peut se dégrader avec le temps. En particulier, le dépolissage peut être abîmé lors de chocs qui se traduisent par des défauts à la surface du support entraînant la formation d'artefacts sur les images acquises par le capteur 16.

La figure 6 représente sous la forme d'un schéma par blocs un exemple de procédé de fabrication d'un support diffusant selon l'invention. Dans le présent exemple de réalisation, on impose à la fois les propriétés de diffusion et d'absorption du support. Les propriétés de diffusion du support sont établies par l'ajout de particules diffusantes, par exemple des particules de dioxyde de titane, à un matériau initialement non absorbant et non diffusant. Les propriétés d'absorption du support sont établies par l'ajout d'une encre au matériau initial, par exemple une encre commercialisée par la société Dabe sous l'appellation Encre de Chine LOTUS. Le matériau initial est, par exemple, une résine époxy non absorbante et non diffusante.

Le procédé commence à l'étape 60 par la préparation de la quantité d'encre et de la quantité de poudre de dioxyde de titane à utiliser. Pour ce faire, on peut utiliser une solution absorbante de référence, contenant de l'encre diluée dans de l'éthanol. En appelant $V_T$ le volume de résine à utiliser, le volume $V_{encre}$ de la solution absorbante de référence à utiliser pour obtenir le coefficient d'absorption $\mu_a(sup)$ souhaité est donné par la relation suivante :

$$V_{encre} = \frac{V_T \cdot \mu_a(sup)}{\mu_a(ref)} \qquad (1)$$

où $\mu_a(ref)$ correspond au coefficient d'absorption de la solution absorbante de référence. Le coefficient $\mu_a(ref)$ est, par exemple, mesuré au préalable à l'aide d'un spectrophotomètre.

En appelant $\mu'_S(sup)$ le coefficient de diffusion réduit du support que l'on souhaite obtenir, la masse m de particules de dioxyde de titane à utiliser est donnée par la relation suivante :

$$m = \frac{V_T \cdot C_{ref} \cdot \mu'_S(sup)}{\mu'_S(ref)} \qquad\qquad (2)$$

où $C_{ref}$ est la concentration de particules de dioxyde de titane dans une solution diffusante de référence et $\mu'_S(ref)$ est le coefficient de diffusion réduit obtenu pour ladite solution de référence. Le coefficient de diffusion réduit peut être mesuré au préalable par des méthodes de réflectivité diffuse ou par des méthodes à résolution temporelle, par exemples les méthodes décrites dans la publication de S. L. Jacques intitulée "Time-resolved reflectance spectroscopy in turbid tissues" (IEEE Transactions on biomedical engineering. 36, 1155-1161 (1989)).

A titre d'exemple, on considère une solution absorbante de référence ayant une concentration d'encre de 1 % dont le coefficient d'absorption $\mu_a(ref)$ pour la longueur d'onde de 633 nm est de l'ordre de 30 cm$^{-1}$ et une solution diffusante de référence ayant une concentration $C_{ref}$ de dioxyde de titane de 10 mg/ml et pour laquelle le coefficient de diffusion réduit $\mu'_S(ref)$ pour la longueur d'onde de 633 nanomètres est de l'ordre de 74 cm$^{-1}$. Pour un volume de résine $V_T$ de 500 ml, et pour obtenir un coefficient d'absorption $\mu_a(sup)$ de 0,2 cm$^{-1}$ et un coefficient de diffusion réduite $\mu'_S(sup)$ de 10 cm$^{-1}$ pour la longueur d'onde de 633 nanomètres, les relations (1) et (2) indiquent qu'il faut utiliser un volume d'encre $V_{encre}$ de 3,3 ml et une masse de dioxyde de titane m de 0,68 g.

A l'étape 60, on prélève le volume $V_{encre}$ de la solution absorbante de référence et on met le volume prélevé dans une fiole. La masse m de dioxyde de titane est pesée et versée dans la fiole. L'ensemble peut être placé dans un bain à ultrasons pour favoriser l'élimination d'éventuels agrégats. Le procédé se poursuit à l'étape 62.

A l'étape 62, on mélange la solution contenant l'encre et le dioxyde de titane au volume $V_T$ de résine. Une possibilité consiste dans le cas d'une résine constituée d'une base et d'un catalyseur, à mélanger en premier lieu la base et le catalyseur et à attendre un peu avant d'ajouter la solution contenant l'encre et le dioxyde de titane. Une autre possibilité consiste à incorporer la solution contenant l'encre et le dioxyde de titane à la base et à ajouter le catalyseur de la résine par la suite. La fiole contenant l'encre et le dioxyde de titane peut être rincée avec de l'éthanol. L'enceinte contenant le mélange résine-encre-dioxyde de titane est secouée pour homogénéiser l'ensemble et peut être placée dans un bain à ultrasons. Le procédé se poursuit à l'étape 64.

A l'étape 64, le mélange résine-encre-dioxyde de titane est placé dans un moule adapté à la forme souhaitée du support. On peut laisser polymériser la résine à température ambiante. Après refroidissement et démoulage, une étape finale d'usinage du support peut éventuellement être prévue.

**Revendications**

1. Dispositif optique (49 ; 54) destiné à l'analyse de la lumière diffuse émise par un milieu diffusant (18), comprenant :

   au moins une source lumineuse (14) à distance du milieu diffusant et adaptée à fournir un faisceau lumineux incident (15) destiné à éclairer le milieu diffusant ;
   au moins un capteur (16) adapté à détecter un rayonnement émis par le milieu diffusant ;
   un support (50 ; 56) du milieu diffusant au moins partiellement non absorbant pour le faisceau lumineux incident et le rayonnement diffus,
   **caractérisé en ce que** tout ou partie du support est constitué d'un matériau diffusant dont le coefficient de diffusion réduit est supérieur à 0,1 cm$^{-1}$ et inférieur à 700 cm$^{-1}$.

2. Dispositif optique selon la revendication 1, dans lequel le coefficient de diffusion réduit du support (50 ; 56) est compris entre 1 et 700 cm$^{-1}$.

3. Dispositif optique selon la revendication 1 ou 2, dans lequel ledit matériau a un coefficient d'absorption compris entre 0.01 et 300 cm$^{-1}$.

4. Dispositif optique selon l'une quelconque des revendications précédentes, dans lequel le matériau diffusant du support est sélectionné pour avoir des propriétés optiques de diffusion et d'absorption voisines de celles du milieu diffusant à analyser.

**5.** Dispositif optique selon l'une quelconque des revendications précédentes, dans lequel le support (50 ; 56) comprend au moins une paroi destinée à être située entre la source et le milieu diffusant et/ou entre le milieu diffusant et le capteur (16).

**6.** Dispositif selon l'une quelconque des revendications précédentes, dans lequel le support (50 ; 56) a la forme d'un berceau destiné à recevoir le milieu diffusant (18).

**7.** Support (50 ; 56) d'un milieu diffusant (18) adapté à un dispositif optique destiné à l'analyse du milieu diffusant, **caractérisé en ce qu'**il est constitué, tout ou partie, d'un matériau diffusant dont le coefficient de diffusion réduit est supérieur à 0,1 cm$^{-1}$ et inférieur à 700 cm$^{-1}$.

**8.** Utilisation du dispositif optique (49 ; 54) selon l'une quelconque des revendications 1 à 6, pour réaliser par tomographie une cartographie à trois dimensions du coefficient d'absorption et/ou du coefficient de diffusion réduit du milieu diffusant (18).

**9.** Utilisation du dispositif optique (49 ; 54) selon l'une quelconque des revendications 1 à 6, le faisceau lumineux incident (15) étant à une première longueur d'onde, l'utilisation consistant à placer dans le milieu diffusant (18) des fluorophores sensibles à la première longueur d'onde et émettant le rayonnement diffus à une seconde longueur d'onde, le capteur fournissant des images utilisées pour déterminer des propriétés des fluorophores et/ou des propriétés du milieu diffusant.

**10.** Procédé de fabrication d'un support (50 ; 56) selon la revendication 7, comprenant les étapes suivante :

préparer une poudre d'un matériau diffusant ;
mélanger la poudre à une solution d'un matériau sensiblement non absorbant, non diffusant et durcissable ; et
injecter le mélange obtenu dans un moule pour former le support.

**11.** Procédé selon la revendication 10, comprenant en outre l'étape consistant à mélanger à la poudre ou à la solution une solution supplémentaire d'un matériau absorbant.

## Fig 1

Analyseur 17

Capteur 16

10

19

20

24

15 18 22

14

## Fig 2

14
Laser

15

30
32
18
34
38

19
16
12

Capteur

Analyseur 17

## Fig 3

46 48 18

44 30

Laser 32

40
14 42

34 42

15

19
16

Capteur

Analyseur 17

Fig 4

Fig 5

Fig 6

Europäisches Patentamt
European Patent Office
Office européen des brevets

**RAPPORT DE RECHERCHE EUROPEENNE**

Numéro de la demande

EP 09 15 8148

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2007/057798 A (KONINKL PHILIPS ELECTRONICS NV [NL]; VAN DER MARK MARTINUS B [NL]; VAN) 24 mai 2007 (2007-05-24) * page 4, ligne 12-20 * * page 5, ligne 26 - page 6, ligne 19 * * page 9, ligne 28 - page 10, ligne 14 * | 1-9 | INV. G01N21/03 G01N21/47 G01N21/64 A61B5/00 |
| Y | ----- | 10,11 | |
| Y | WO 99/40411 A (DAEDALUS I LLC [US]) 12 août 1999 (1999-08-12) * page 8, ligne 22-36 * | 10,11 | |
| X | ----- WO 02/42824 A (P A L M MICROLASER TECHNOLOGIE [DE]; SCHUETZE KARIN [DE]; SCHUETZE RAI) 30 mai 2002 (2002-05-30) * page 1, ligne 12-16,32,33 * * page 4, ligne 16-23 * * page 7, ligne 9-32 * * page 9, ligne 13-16,24-32 * * page 10, ligne 1-9 * * page 12, ligne 9-13 * * page 13, ligne 2-14 * * page 15, ligne 18-32 * | 1-6,8-10 | |
| X | ----- WO 02/095476 A (MEDICAL RES COUNCIL [GB]; SHARPE JAMES ALEXANDER [GB]; PERRY PAUL ERNE) 28 novembre 2002 (2002-11-28) * page 5, ligne 17 - page 6, ligne 15 * | 1-8 | DOMAINES TECHNIQUES RECHERCHES (IPC) G01N A61B |
| X | ----- SHARPE J: "Optical Projection Tomography" ANNU. REV. BIOMED. ENG., vol. 6, 9 avril 2004 (2004-04-09), pages 209-228, XP002507150 USA * page 218, ligne 35-38; figures 1-3 * ----- -/-- | 1-8 | |

Le présent rapport a été établi pour toutes les revendications

| Lieu de la recherche | Date d'achèvement de la recherche | Examinateur |
|---|---|---|
| Berlin | 14 juillet 2009 | Navas Montero, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

EP 2 110 657 A1

Europäisches
Patentamt

European
Patent Office

Office européen
des brevets

**RAPPORT DE RECHERCHE EUROPEENNE**

Numéro de la demande

EP 09 15 8148

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DINTEN J.-M. ET AL:  "Performance of different reflectance and diffuse optical imaging tomographic approaches in fluorescence molecular imaging of small animals" PROCEEDINGS OF SPIE. MEDICAL IMAGING 2006: PHYSICS OF MEDICAL IMAGING, vol. 6142, 2006, pages 614215-1-614215-10, XP002507356 USA * figures 5,6 *  ----- | 1,7 | |
| | | | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| Berlin | 14 juillet 2009 | Navas Montero, E |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

............................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 15 8148

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-07-2009

| Document brevet cité au rapport de recherche | | Date de publication | Membre(s) de la famille de brevet(s) | | Date de publication |
|---|---|---|---|---|---|
| WO 2007057798 | A | 24-05-2007 | CN | 101309632 A | 19-11-2008 |
| | | | EP | 1951107 A2 | 06-08-2008 |
| | | | JP | 2009516193 T | 16-04-2009 |
| | | | US | 2009046291 A1 | 19-02-2009 |
| WO 9940411 | A | 12-08-1999 | AT | 356985 T | 15-04-2007 |
| | | | AU | 2661899 A | 23-08-1999 |
| | | | EP | 0980518 A1 | 23-02-2000 |
| WO 0242824 | A | 30-05-2002 | AU | 1597802 A | 03-06-2002 |
| | | | DE | 10058316 A1 | 13-06-2002 |
| WO 02095476 | A | 28-11-2002 | AT | 305623 T | 15-10-2005 |
| | | | CA | 2445780 A1 | 28-11-2002 |
| | | | DE | 60206388 T2 | 11-05-2006 |
| | | | DE | 60223728 T2 | 30-10-2008 |
| | | | DK | 1530073 T3 | 25-03-2008 |
| | | | EP | 1410090 A2 | 21-04-2004 |
| | | | EP | 1530073 A1 | 11-05-2005 |
| | | | ES | 2248548 T3 | 16-03-2006 |
| | | | ES | 2294583 T3 | 01-04-2008 |
| | | | HK | 1067412 A1 | 27-01-2006 |
| | | | JP | 2004531729 T | 14-10-2004 |
| | | | PT | 1530073 E | 12-12-2007 |
| | | | US | 2004207840 A1 | 21-10-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Littérature non-brevet citée dans la description**

- **S. L. Jacques.** Time-resolved reflectance spectroscopy in turbid tissues. *IEEE Transactions on biomedical engineering,* 1989, vol. 36, 1155-1161